# EUROPÄISCHE PATENTSCHRIFT

(11) **EP 1 277 403 B1**
(45) Veröffentlichungstag und Bekanntmachung des Hinweises auf die Patenterteilung: **21.09.2005**
(21) Anmeldenummer: 02015840.8
(22) Anmeldetag: 16.07.2002
(51) Int. Cl.: A01N 33/12, A01N 47/44, A01N 33/04, A01N 25/30, A61L 2/18

(54) **Desinfektionsmittelkonzentrate auf Basis quaternärer Ammoniumverbindungen sowie die Verwendung derselben zur maschinellen Instrumentendesinfektion**
Disinfectant concentrate based on quaternary ammonium compounds and its use for automated instrument disinfection
Concentré désinfectant et son utilisation dans la désinfection des instruments par machine

(30) Priorität: 17.07.2001 DE 10134723
(43) Veröffentlichungstag der Anmeldung: 22.01.2003
(73) Patentinhaber: Bode Chemie GmbH & Co., 22525 Hamburg (DE)
(72) Erfinder: Fehling, Thomas, 22305 Hamburg (DE); Knieler, Roland, Dr., 21227 Bendestorf (DE); Bloss, Richard, Dr., 25462 Rellingen (DE)
(74) Vertreter: Rabanus, Birgit

(56) Entgegenhaltungen:
- EP-A- 0 343 605
- EP-A- 0 639 636
- EP-A- 1 195 091
- WO-A-00/31224

## Beschreibung

Die vorliegende Erfindung betrifft Desinfektionsmittelkonzentrate und ihre Verwendung zur maschinellen Instrumentendesinfektion.

Als Desinfektionsmittel zur Desinfektion von Instrumenten und Wäsche sind eine Vielzahl mikrobizid wirksamer chemischer Substanzen bzw. Gemische dieser Substanzen an sich bekannt. Mikrobizide Substanzen sind im allgemeinen gegen das übliche Spektrum von Keimen, wie beispielsweise grampositive Bakterien, gramnegative Bakterien, Mykobakterien, Hefen, Pilze, Viren und dergleichen, mehr oder weniger wirksam, so daß man üblicherweise eine ausreichende Desinfektion durch geeignete Wirkstoffkombinationen erzielen kann.

EP 1 195 091 A1 offenbart Desinfektionsmittelformulierungen, welche neben einem Gehalt an Wirkstoffen gewählt aus den Gruppen quaternäre Ammoniumverbindungen, Alkylamine, Alkylthiouroniumsalze, α, ω-Alkylendithiouroniumsalze und/oder Gemische derselben a) 1 bis 50 Gew.-% mindestens einer Komplexbildnerkomponente, b) 1 bis 50 Gew.-% mindestens eines nichtionischen Tensids und c) 0,1 bis 10 Gew.-% eines hydrophoben Schaumregulators, insbesondere N-Alkyl-Isononansäureamid, enthalten, wobei die Prozentangaben jeweils auf das Gesamtgewicht der Formulierungen bezogen sind. Die Desinfektionsmittel werden zur maschinellen Intrumentendesinfektion eingesetzt. EP 1 195 091 A1 wurde nach dem von der vorliegenden Anmeldung beanspruchten Prioritätsdatum veröffentlicht und gehört somit nur gemäß Artikel 54(3) EPÜ zum Stand der Technik.

EP 0 343 605 A1 offenbart ein flüssiges aldehydfreies tuberkulozides Desinfektionsmittel, gekennzeichnet durch N,N-Bis-(3-aminopropyl)-laurylamin als wirksame Komponente. Das Desinfektionsmittel kann zusätzlich mindestens ein Tensid enthalten. Vorzugsweise werden anionische, nichtionische oder amphotere Tenside sowie verträgliche Kombinationen daraus eingesetzt. Das Mittel kann weiterhin Komplexbildner enthalten, bevorzugt Ethylendiamintetraessigsäure oder Nitriloessigsäure und deren Salze. Das Mittel kann noch weitere Zusätze wie quaternäre Ammoniumverbindungen und zusätzliche Wirkstoffe wie Guanidinderivate oder Biguanide enthalten.

WO 00/31224 A1 offenbart ein wässriges Reinigungsmittel für harte Oberflächen, enthaltend Tensid und diquaternäres Polysiloxan, dadurch gekennzeichnet, dass es, bezogen auf die Gesamtmenge an Tensid und diquaternären Polysiloxan, mindestens 1 Gew.-% anionisches Tensid enthält. Das Mittel kann als Tensidkomponente anionische, nichtionische, amphotere oder kationische Tenside bzw. Tensidgemische aus einer, mehreren oder allen dieser Tensidklassen enthalten. Bevorzugte Ausführungsformen enthalten anionische und nichtionische Tenside nebeneinander. WO 00/31224 A1 beschreibt ebenfalls die Verwendung von diquaternärem Polysiloxan in einem flüssigen Reinigungsmittel für harte Oberflächen zur Verkürzung der Trocknungszeit und ein Verfahren zur Verkürzung der Trockungszeit einer mit einem flüssigen Reinigungsmittel behandelten harten Oberfläche. Dieses Verfahren ist dadurch gekennzeichnet, dass die Oberfläche mit einem diquaternäres Polysiloxan enthaltenden flüssigen Reinigungsmittel behandelt wird.

Der Stand der Technik kennt zur Desinfektion von medizinisch-chirurgischen Instrumenten eine Reihe von Wirkstoffen, insbesondere Aldehyde, wie beispielsweise Formaldehyd oder Glutaraldehyd, quaternäre Ammoniumverbindungen und langkettige Amine sowie Phenole. Allerdings weisen die genannten Verbindungen einige Nachteile auf:

Aldehyde fixieren Reste von Blut und Eiweiß durch chemische Reaktion an den zu desinfizierenden Gegenständen, so daß diese nach der Desinfektion schwer zu reinigen sind.

Quaternäre Ammoniumverbindungen und langkettige Amine werden häufig in der manuellen Instrumentendesinfektion verwendet. Im Vergleich zu den Aldehyden ist der Geruch dieser Verbindungen deutlich weniger unangenehm. Eine chemische Reaktion mit Eiweißen erfolgt nicht, jedoch kommt es zu einer physikalischen Fällung von Eiweißen, die zum Teil durch geschickte Kombination mit Tensiden kompensiert werden kann. Für die maschinelle Instrumentendesinfektion sind die quaternären Ammoniumverbindungen allerdings nicht geeignet, weil es infolge der Turbulenzen in der Reinigungsmaschine zu einer starken, unerwünschten Schaumbildung kommt. Ein weiterer entscheidender Nachteil ist das eingeengte Wirkungsspektrum quaternärer Ammoniumverbindungen, die nicht gegen gramnegative Bakterien wirken.

Auch Alkylamine, wie beispielsweise N,N-Bis-(3-aminopropyl)-laurylamin, sind prinzipiell zur Instrumentendesinfektion geeignet. Allerdings haben auch sie den Nachteil, in einer Reinigungsmaschine stark zu schäumen, weshalb auch sie sich für die chemo-thermische Desinfektion bislang nicht durchgesetzt haben.

Phenole sind vor allem wegen ihres Geruches, ihrer geringen Wirksamkeit gegen den Poliovirus, ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.

Aufgabe der Erfindung war es daher, Desinfektionsmittel zur maschinellen Instrumentendesinfektion zu finden, welche die Nachteile des Standes der Technik nicht aufweisen, welche also eine gute Reinigungsleistung bei zugleich geringer Toxizität zeigen, weder Eiweiß noch Blut an den zu desinfizierenden Gegenständen fixieren, keinen unangenehmen Geruch haben und insbesondere in Wasch- und Desinfektionsautomaten für chirurgische Instrumente keine störende Schaumbildung verursachen. Ferner sollte das Desinfektionsmittel möglichst aldehydfrei sein.

Zwar ließe sich durch den Einsatz von wasserunlöslichen Entschäumern - z. B. auf Silikonbasis - die Schaumentwicklung der tensidischen Wirkstoffe (quaternäre Ammoniumverbindungen oder Alkylamine) dämpfen. Allerdings wären hierzu für dieses Einsatzgebiet so große Mengen notwendig, daß dies keine Lösung des Problems darstellt.

Ein weiterer Nachteil derartiger Entschäumer des Standes der Technik ist, daß diese innerhalb eines chemo-thermischen Desinfektionsprogrammes einen Film auf den Instrumenten und dem Maschineninnenraum hinterlassen (sog. Build up"-Effekt), welcher sich nur schwer wieder entfernen läßt und zudem zu Materialverträglichkeitsproblemen mit den empfindlichen thermolabilen Instrumenten und darüberhinaus zu einer Beeinträchtigung der Wirksamkeit führen kann, da derartige Silkionverbindungen einen vergleichsweise guten Nährboden für Mikroorganismen darstellen.

Auf der anderen Seite besteht aber ein grosser Bedarf an Desinfektionsmitteln für die chemo-thermische Instrumentendesinfektion, die einerseits die Vorteile aldehydfreier Wirkstoffe aufweisen, aber andererseits deren Nachteile nicht zeigen.

Es war überraschend und für den Fachmann nicht vorauszusehen, daß Desinfektionsmittelformulierungen, welche neben einem Gehalt an mindestens einem Wirkstoff gewählt aus der Gruppe, welche gebildet wird aus quaternären Ammoniumverbindungen, Alkylaminen, Biguaniden und polymeren Biguaniden
a) 1 bis 50 Gew.-% mindestens einer Komplexbildnerkomponente,
b) 1 bis 50 Gew.-% mindestens eines Lösungsvermittlers und
c) 1 bis 50 Gew.-% mindestens eines wasserlöslichen, kationischen Silikon-tensides
enthalten,
wobei die Prozentangaben jeweils auf das Gesamtgewicht der Formulierungen bezogen sind,
den geschilderten Nachteilen des Standes der Technik Abhilfe schaffen würden.

Die erfindungsgemäßen Desinfektionsmittelformulierungen (in folgenden auch "Desinfektionsmittelkonzentrate" genannt) werden nach Verdünnen mit Wasser - in Form einer sogenannten "Gebrauchslösung" - als Desinfektionsmittel verwendet.

Desinfektionsmittelformulierungen im Sinne der vorliegenden Erfindung sind besonders geeignet zum Desinfizieren von Instrumenten, insbesondere zur maschinellen Instrumentenaufbereitung.

Die bakterizide und fungizide Wirkung der erfindungsgemäßen Formulierungen ist ausgesprochen gut. Von Vorteil für ihre Anwendung im Bereich der maschinellen Instrumentenaufbereitung ist insbesondere ihre inaktivierende Wirkung auf das Hepatitis B-Virus. Das Hepatitis B-Oberflächenantigen wird bereits nach einer Einwirkungszeit von 5 Minuten bei der maschinellen Anwendung zerstört.

Überraschenderweise bilden die erfindungsgemäßen Formulierungen - insbesondere in Form einer Gebrauchslösung - im Gegensatz zum Verhalten der Einzelwirkstoffe selbst bei starker Turbulenz nur eine sehr geringe Schaummenge, wodurch ein Einsatz dieser Wirkstoffe für die maschinelle chemo-thermische Instrumentendesinfektion möglich wird, ohne daß wasserunlösliche Silikonentschäumer eingesetzt werden müssen.

Ferner verursachen Desinfektionsmittelformulierungen gemäß der vorliegenden Erfindung in ihrer Umgebung keine bzw. nur eine geringe Korrosion. Dies ist naturgemäß insbesondere für die Instrumentendesinfektion von Vorteil, da sowohl die zu desinfizierenden Gegenstände als auch die verwendeten Wasch- und Desinfektionsautomaten im allgemeinen aus Metall gefertigt sind bzw. Metallteile enthalten.

Die Desinfektionsmittelkonzentrate stellen klare, einphasige Mischungen dar, welche in einem Temperaturbereich von 5 bis 50 °C völlig stabil sind. Die wäßrige Gebrauchslösung hingegen, welche vorteilhaft beispielsweise 0,2 bis 2,0 Gew.-% an Konzentrat enthält, kann in einem Temperaturbereich von 20 bis 60 °C eine trübe Emulsion darstellen, die äußerst schaumarm und somit ganz besonders für den maschinellen Einsatzbereich geeignet ist.

Es war in keiner Weise vorhersehbar, daß die Kombination der tensidischen Wirkstoffe mit den erfindungsgemäßen Hilfsstoffen den oben beschriebenen Effekt ermöglichen würden.

Erfindungsgemäß vorteilhafte Komplexbildnerkomponenten sind organische Phosphonatderivate, wie z. B. Hydroxyethylamindimethylenphosphonsäure, Hydroxyethan-1,1-diphosphonsäure, 2-Phosphonobutan-tricarbonsäure-1,2,4, Phosphonohydroxyessig-säure u.a.. Es können aber auch andere Komplexbildner, z. B. Nitrilotriessigsäure und ihr Natriumsalz, verwendet werden oder Kombinationen dieser Komplexbildner. Besonders bevorzugter Komplexbildner im Sinne der vorliegenden Erfindung ist Hydroxyethylamindimethylenphosphonsäure.

Es ist besonders bevorzugt, den Gehalt an dem oder den Komplexbildnerkomponenten in der Desinfektionsmittelformulierung aus dem Bereich zwischen 1 und 50 Gew.-%, insbesondere von 1 bis 10 Gew.-%, bezogen auf das Gesamtgewicht der Formulierung, zu wählen.

Erfindungsgemäß vorteilhafte Lösungsvermittler sind beispielsweise aliphatische Alkohole, Glykole und/oder Polyglykole, wie Dipropylenglykol, Monoethylenglykol, Diethylenglykol, Triethylenglykol, 1,2-Propylenglykol und Copolymerisate von Ethylenoxid und Propylenoxid. Besonders bevorzugt Lösungsvermittler im Sinne der vorliegenden Erfindung ist Dipropylenglykol.

Es wird bevorzugt, den Gehalt an einem oder mehreren Lösungsvermittlern zwischen 1 und 50 Gew.-%, insbesondere zwischen 1 und 10 Gew.-%, bezogen auf das Gesamtgewicht der Zubereitungen, zu wählen.

Erfindungsgemäß bevorzugte Silikontenside sind kationische, diquaternäre Polydimethylsiloxane, sog. "Silicon-Quats" (z. B. Tegopren® 6922 von Th. Goldschmidt), mit einen Gehalt an organischen, quaternären Stickstoffgruppen von 0,1 bis 0,8 Gew.-%, welche trotz ihrer hydrophoben Eigenschaften voll wasserlöslich sind und nicht zu einem sog. "Build up"-Effekt führen.

Es ist besonders bevorzugt, den Gehalt an dem oder den diquaternären Polydimethylsiloxanen in der Desinfektionsmittelformulierung aus dem Bereich von 1 bis 50 Gew.-%, insbesondere zwischen 1 und 10 Gew.-%, bezogen auf das Gesamtgewicht der Formulierung, zu wählen.

Es ist erfindungsgemäß bevorzugt, die quaternäre(n) Ammoniumverbindung(en) aus der Gruppe Benzalkoniumchlorid, N-Dodecyl-N,N-dimethyl-ethylbenzylammoniumchlorid, Dioctyldimethylammoniumchlorid, Didecyldimethylammoniumchlorid, Decylisononyldimethylammoniumchlorid und Didecylmethylpolyoxyethylammoniumpropionat zu wählen.

Es ist ferner bevorzugt, den Gehalt an der oder den quaternären Ammoniumverbindung(en) in der Desinfektionsmittelformulierung aus dem Bereich von 0,5 bis 50 Gew.-%, vorzugsweise zwischen 0,5 und 10 Gew.-%, bezogen auf das Gesamtgewicht der Formulierung, zu wählen. Zu bevorzugen ist hierbei eine Kombination von N-Dodecyl-N,N-dimethyl-ethylbenzyl-ammoniumchlorid und Dioctyldimethylammoniumchlorid.

Sollen als Wirkstoff ein oder mehrere Alkylamine eingesetzt werden, so sind diese besonders vorteilhaft aus der Gruppe der Alkylamine zu wählen, welche sich durch die folgende Strukturformel auszeichnen: 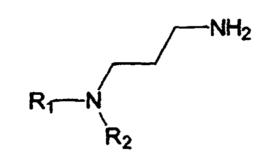 worin R₁ eine verzweigte oder unverzweigte, gesättigte oder ungesättigte Alkylgruppe mit 8 bis 16 Kohlenstoffatomen und R₂ eine Aminoalkylgruppe mit 1 bis 3 Kohlenstoffatomen, vorzugsweise (CH₂)₃NH₂, oder Wasserstoff darstellen. Vorteilhaft wird der Gehalt an diesen Alkylbispropylentriamin(en) und/oder Alkylpropylendiamin(en) in der Desinfektionsmittelformulierung aus dem Bereich von 0,5 bis 50 Gew.-%, vorzugsweise von 0,5 bis 5 Gew.-% gewählt, jeweils bezogen auf das Gesamtgewicht der Formulierung.

Es ist erfindungsgemäß bevorzugt, die Biguanide aus der Gruppe der polymeren Biguanide wie z. B. Polyhexamethylen-biguanid-Hydrochlorid, allgemein auch als Polihexanid bekannt, zu wählen. Es ist ferner bevorzugt, den Gehalt an der oder den Biguaniden in der Desinfektionsmittelformulierung aus dem Bereich von 0,5 bis 50 Gew.-%, vorzugsweise zwischen 0,5 und 5 Gew.-%, bezogen auf das Gesamtgewicht der Formulierung, zu wählen.

Die genannten Wirkstoffe können sowohl einzeln als auch in beliebigen Mischungen zueinander eingesetzt werden.

Erfindungsgemäß vorteilhaft wird der pH-Wert des Desinfektionsmittelkonzentrates auf Werte zwischen 2 bis 12 und vorzugsweise 5 bis 9 eingestellt. Zur pH-Einstellung können anorganische oder organische Säuren und Basen verwendet werden, wie beispielsweise o. g. Phosphonsäurederivate, welche zudem das Kalkbindevermögen der Anwendungslösung erhöht.

Ferner können die erfindungsgemäßen Zubereitungen vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 5 Gew.-%, bezogen auf das Gesamtgewicht der Zubereitungen, zu wählen.

Zusätzlich zu den vorstehend genannten Komponenten können die erfindungsgemäßen Zubereitungen andere übliche Hilfsstoffe, wie z. B. nichtionische Tenside, Komplexbildner, Sequestiermittel, pH-Regulatoren, Lösevermittler und Schaumregulatoren enthalten. Diese dienen der Verbesserung der Gebrauchseigenschaften der Desinfektionsmittelformulierung wie z. B. der Reinigungsleistung, der Wasserenthärtung, der Schaumunterdrückung und der Stabilität.

Zusätzlich zu den vorstehend genannten Komponenten können die erfindungsgemäßen Zubereitungen für derartige Zubereitungen übliche Konservierungsstoffe, Farbstoffe und Duftstoffe enthalten. Diese tragen in der Mehrzahl der Fälle nicht zur desinfizierenden Wirkung bei, sondern dienen der Lagerbarkeit sowie ästhetischen Zwecken. Es ist jedoch auch möglich, solche Komponenten zu verwenden, die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die folgenden Beispiele dienen dazu, die Erfindungen zu beschreiben, selbstverständlich ohne daß beabsichtigt ist, die Erfindungen auf diese Beispiele zu beschränken. Alle Prozentanteile sind, soweit nicht anders angegeben, auf das Gesamtgewicht der Zubereitungen bezogen.

### Beispiele:

Angaben in Gew.-%

### Beispiel 1:

| | |
|---|---|
| Dipropylenglykol | 6,0 % |
| Polihexanid | 1,6 % |
| Dioctyldimethylammoniumchlorid | 4,0 % |
| N-Dodecyl-N,N-dimethyl-ethylbenzylammoniumchlorid | 1,0 % |
| Hydroxyethylamindimethylenphosphonsäure | 3,0 % |
| Diquaternäres Polydimethylsiloxan | 1,5 % |
| Korrosioninhibitoren | 0,2 % |
| Rest: Hilfsstoffe, Wasser | |

### Beispiel 2:

| | |
|---|---|
| Dipropylenglykol | 6,0 % |
| Dioctyldimethylammoniumchlorid | 4,5 % |
| N-Dodecyl-N,N-dimethyl-ethylbenzylammoniumchlorid | 1,5 % |
| Hydroxyethylamindimethylenphosphonsäure | 3,0 % |
| Diquatemäres Polydimethylsiloxan | 1,5 % |
| Korrosioninhibitoren | 0,2 % |
| Rest: Hilfsstoffe, Wasser | |

### Beispiel 3:

| | |
|---|---|
| Dipropylenglykol | 6,0 % |
| Alkylbispropylentriamin | 0,9 % |
| Dioctyldimethylammoniumchlorid | 4,0 % |
| N-Dodecyl-N,N-dimethyl-ethylbenzylammoniumchlorid | 1,0 % |
| Hydroxyethylamindimethylenphosphonsäure | 3,0 % |
| Diquaternäres Polydimethylsiloxan | 1,5 % |
| Korrosioninhibitoren | 0,2 % |
| Rest: Hilfsstoffe, Wasser | |

Bei den Beispielen handelt es um Desinfektionsmittelkonzentrate, welche vor ihrer Anwendung mit Wasser verdünnt werden.

Alle Rezepturen sind stabil und bilden in wässriger Verdünnung eine schaumarme Emulsion, welche für einen maschinellen Einsatz geeignet ist.

## Patentansprüche

1. Desinfektionsmittelformulierungen, welche neben einem Gehalt an an mindestens einem Wirkstoff gewählt aus der Gruppe, welche gebildet wird aus quaternären Ammoniumverbindungen, Alkylaminen, Biguaniden und polymeren Biguaniden
a) 1 bis 50 Gew.-% mindestens einer Komplexbildnerkomponente,
b) 1 bis 50 Gew.-% mindestens eines Lösevermittlers und
c) 1 bis 50 Gew.-% mindestens eines wasserlöslichen, kationischen Silikon-tensides
enthalten,
wobei die Prozentangaben jeweils auf das Gesamtgewicht der Formulierungen bezogen sind.

2. Desinfektionsmittelformulierung nach Anspruch 1, **dadurch gekennzeichnet, daß** zusätzlich Tenside, Lösevermittler, Korrosionsinhibitoren, Schaumregulatoren, pH-Regulatoren, Komplexbildner, Sequestiermittel und/oder weitere Hilfs-, Zusatz- und/oder Wirkstoffe enthalten sind.

3. Desinfektionsmittelformulierung nach Anspruch 1 oder 2, **dadurch gekennzeichnet, daß** es sich um Konzentrate handelt, die vor der Verwendung mit Wasser zu einer Gebrauchslösung verdünnt werden.

4. Desinfektionsmittelformulierung nach einem der Ansprüche 1 bis 3, **dadurch gekennzeichnet, daß** die Komplexbildnerkomponente(n) aus der Gruppe organische Phosphonatderivate und ihrer Salze gewählt werden, insbesondere Hydroxyethylamindimethylenphosphonsäure.

5. Desinfektionsmittelformulierung nach einem der Ansprüche 1 bis 4, **dadurch gekennzeichnet, daß** der oder die Lösevermittler aus der Gruppe der aliphatischen Alkohole, Glykole und/oder Polyglykole gewählt werden, wie z. B. Dipropylenglykol, Monoethylenglykol, Diethylenglykol, Triethylenglykol, 1,2-Propylenglykol und Copolymerisaten von Ethylenoxid und Propylenoxid, vorzugsweise jedoch Dipropylenglykol.

6. Desinfektionsmittelformulierung nach einem der Ansprüche 1 bis 5, **dadurch gekennzeichnet, daß** das oder die wasserlöslichen, kationischen Silikontenside aus der Gruppe der diquaternären Polydimethylsiloxane, insbesondere solche mit einem Gehalt an organischen, quaternären Stickstoffgruppen zwischen 0,1 bis 0,8 Gew.-%, gewählt werden.

7. Desinfektionsmittelformulierung nach einem der Ansprüche 1 bis 6, welche als Konzentrat vorliegt, **dadurch gekennzeichnet, daß** einerseits das Konzentrat in einem Temperaturbereich von 5 bis 50 °C eine klare Mischung darstellt und daß andererseits dessen wässrige Verdünnung, welche 0,2 bis 2,0 Gew.-% an Konzentrat enthält, in einem Temperaturbereich von 20 bis 60° C eine trübe, mehrphasige Emulsion darstellen kann, welche sich durch ihre schwachschäumende Eigenschaft auszeichnet.

8. Verwendung von Gebrauchslösungen nach Anspruch 3 als Desinfektionsmittel für medizinisch-chirurgische Instrumente in Wasch- und/oder Desinfektionsautomaten.

9. Verwendung von Gebrauchslösungen nach Anspruch 3 als Desinfektionsmittel für die chemo-thermische Aufbereitung flexibler Endoskope.

10. Desinfektionsmittelformulierung nach einem der Ansprüche 1 bis 7, welche sich aus folgenden Inhaltsstoffen zusammensetzt:
| | Gew.-% |
|---|---|
| Dipropylenglykol | 1 - 10 |
| Polihexanid | 0,5 - 5 |
| Dioctyldimethylammoniumchlorid | 0,5 - 10 |
| N-Dodecyl-N,N-dimethyl-ethylbenzylammoniumchlorid | 0,5 - 10 |
| Hydroxyethylamindimethylenphosphonsäure | 1 - 10 |
| Diquaternäres Polydimethylsiloxan | 1 - 10 |
| Rest: Hilfsstoffe, Wasser | |

11. Desinfektionsmittelformulierung nach einem der Ansprüche 1 bis 7, welche sich aus folgenden Inhaltsstoffen zusammensetzt:
| | Gew.-% |
|---|---|
| Dipropylenglykol | 1 - 10 |
| Dioctyldimethylammoniumchlorid | 0,5 - 10 |
| N-Dodecyl-N,N-dimethyl-ethylbenzylammoniumchlorid | 0,5 - 10 |
| Hydroxyethylamindimethylenphosphonsäure | 1 - 10 |
| Diquaternäres Polydimethylsiloxan | 1 - 10 |
| Rest: Hilfsstoffe, Wasser | |

12. Desinfektionsmittelformulierung nach einem der Ansprüche 1 bis 7, welche sich aus folgenden Inhaltsstoffen zusammensetzt:
| | Gew.-% |
|---|---|
| Dipropylenglykol | 1 - 10 |
| Alkylbispropylentriamin | 0,5 - 5 |
| Dioctyldimethylammoniumchlorid | 0,5 - 10 |
| N-Dodecyl-N,N-dimethyl-ethylbenzylammoniumchlorid | 0,5 - 10 |
| Hydroxyethylamindimethylenphosphonsäure | 1 - 10 |
| Diquaternäres Polydimethylsiloxan | 1 - 10 |
| Rest: Hilfsstoffe, Wasser | |

## Claims

1. Disinfectant formulations which, in addition to a content of at least one active substance selected from the group which is formed from quaternary ammonium compounds, alkylamines, biguanides and polymeric biguanides, contain
a) 1 to 50% by weight of at least one complexing agent component,
b) 1 to 50% by weight of at least one solubilizer and
c) 1 to 50% by weight of at least one water-soluble, cationic silicone surfactant, whereby the percentage figures refer to the total weight of the formulations respectively.

2. Disinfectant formulation according to Claim 1, **characterized** thereby that additional surfactants, solubilizers, corrosion inhibitors, foam regulators, pH-regulators, complexing agents, sequestering agents and/or further auxiliary, additive and/or active substances are contained therein.

3. Disinfectant formulation according to Claim 1 or 2, **characterized** thereby that it involves concentrates which are diluted to an application solution with water before use.

4. Disinfectant formulation according to one of Claims 1 to 3, **characterized** thereby that the complexing agent component(s) are selected from the group of organic phosphate derivatives and salts thereof, in particular hydroxyethylamine dimethylene phosphonic acid.

5. Disinfectant formulation according to one of Claims 1 to 4, **characterized** thereby that the solubilizer or solubilizers are selected from the group of aliphatic alcohols, glycols and/or polyglycols, such as dipropylene glycol, monoethylene glycol, diethylene glycol, triethylene glycol, 1,2 propylene glycol and copolymers of ethylene oxide and propylene oxide, preferably however dipropylene glycol.

6. Disinfectant formulation according to one of Claims 1 to 5, **characterized** thereby that the water-soluble, cationic silicone surfactant or surfactants are selected from the group of diquaternary polydimethylsiloxanes, in particular those such with a content of organic, quaternary nitrogen groups between 0.1 to 0.8% by weight.

7. Disinfectant formulation according to one of claims 1 to 6, which is present as a concentrate **characterized** thereby that, on the one hand, the concentrate represents a clear mixture in a temperature range from 5 to 50 °C while, on the other hand, its aqueous solution containing 0.2 to 2.0% by weight of concentrate can represent a cloudy polyphase emulsion **characterized by** its low-foaming property in a temperature range from 20 to 60° C.

8. Use of application solutions according to Claim 3 as disinfectants for medical-surgical instruments in washing or disinfecting machines.

9. Use of application solutions according to Claim 3 as disinfectants for the chemothermal preparation of flexible endoscopes.

10. Disinfectant formulation according to one of Claims 1 to 7, which is composed of the following ingredients:
| | Weight |
|---|---|
| Dipropylene glycol | 1-10 |
| Polihexanide | 0.5-5 |
| Dioctyldimethylammonium chloride | 0.5-10 |
| N-Dodecyl-N,N-dimethyl-ethylbenzylammonium chloride | 0.5-10 |
| Hydroxyethylamine dimethylene phosphonic acid | 1-10 |
| Diquaternary polydimethylsiloxane | 1-10 |
| Remainder: Auxiliaries, water | |

11. Disinfectant formulation according to one of Claims 1 to 7, which is composed of the following ingredients:
| | Weight % |
|---|---|
| Dipropylene glycol | 1-10 |
| Dioctyldimethylammonium chloride | 0.5-10 |
| N-Dodecyl-N,N-dimethyl-ethylbenzylammonium chloride | 0.5-10 |
| Hydroxyethylamine dimethylene phosphonic acid | 1-10 |
| Diquaternary polydimethylsiloxane | 1-10 |
| Remainder: Auxiliaries, water | |

12. Disinfectant formulation according to one of Claims 1 to 7, which is composed of the following ingredients:
| | Weight % |
|---|---|
| Dipropylene glycol | 1-10 |
| Alkylbispropylene triamine | 0.5-5 |
| Dioctyldimethylammonium chloride | 0.5-10 |
| N-Dodecyl-N,N-dimethyl-ethylbenzylammonium chloride | 0.5-10 |
| Hydroxyethylamine dimethylene phosphonic acid | 1-10 |
| Diquaternary polydimethylsiloxane | 1-10 |
| Remainder: Auxiliaries, water | |

## Revendications

1. Formulations de désinfection, comprenant, outre une teneur en au moins une substance active choisie dans le groupe comprenant des liaisons ammoniums quaternaires, des alkylamines, des biguanides et des biguanides polymères, également
a) 1 à 50 % en poids au moins d'un composant complexant,
b) 1 à 50 % en poids au moins d'un intermédiaire de solubilité et
c) 1 à 50 % en poids au moins d'un tensio-actif de silicone cationique hydrosoluble,
d) où le pourcentage se rapporte au poids total de la formulation.

2. Formulation de désinfection selon la revendication 1, **caractérisée en ce qu'**elle renferme également un tensio-actif, un intermédiaire de solubilité , des inhibiteurs de corrosion, des agents anti-mousse, des régulateurs de pH, des complexants, des séquestrants et / ou d'autres auxiliaires, additifs et / ou substances actives.

3. Formulation de désinfection selon l'une quelconque des revendications 1 ou 2, **caractérisée en ce qu'**elle représente des concentrés à diluer dans l'eau avant utilisation pour obtenir une solution prête à l'emploi.

4. Formulation de désinfection selon l'une quelconque des revendications 1 à 3, **caractérisée en ce que** le(s) complexant(s) soi(en)t choisi(s) dans le groupe comprenant des dérivés de phosphonate organique et leurs sels, et en particulier les acides hydroxyéthylamine-diméthylène-phosphonique.

5. Formulation de désinfection selon l'une quelconque des revendications 1 à 4, **caractérisée en ce que** le ou les intermédiaire(s) de solubilité soient choisis dans le groupe comprenant des alcools aliphatiques, des glycols et / ou des polyglycols, comme par exemple le dipropylèneglycol, le monoéthyléneglycol, le diéthylèneglycol, le triéthylèneglycol, le 1,2-propylèneglykol et les copolymères de l'oxyde d'éthylène et de l'oxyde de propylène, préférentiellement toutefois le dipropylèneglycol.

6. Formulation de désinfection selon l'une quelconque des revendications 1 à 5, **caractérisée en ce que** le(s) tensio-actif(s) de silicone cationiques) hydrosoluble(s) soient choisis dans le groupe comprenant les polydiméthylsiloxanes di-quatemaires, et en particulier ceux présentant une teneur en groupes azote organiques quaternaires comprise entre 0,1 et 0,8 % en poids.

7. Formulation de désinfection selon l'une quelconque des revendications 1 à 6, disponible sous forme de concentré, **caractérisée en ce que**, d'une part, le concentré constitue un mélange clair entre 5 et 50 °C et que, d'autre part, sa dilution aqueuse, qui renferme 0,2 à 2,0 % en poids de concentré, constitue une émulsion à plusieurs phases, trouble entre 20 et 60° C et qui se distingue par ses propriétés faiblement moussantes.

8. Utilisation de solutions prête à l'emploi selon la revendication 3 comme désinfectant d'instruments médicaux et chirurgicaux en automates de lavage et / ou de désinfection.

9. Utilisation de solutions prête à l'emploi selon la revendication 3 comme désinfectant pour la préparation chimico-thermique d'endoscopes souples.

10. Formulation de désinfection selon l'une quelconque des revendications 1 à 7, composée des substances suivantes :
| | % en |
|---|---|
| Dipropylène glycol | 1-10 |
| Polihexanide | 0,5-5 |
| Chlorure de dioctyl-diméthyl-ammonium | 0,5-10 |
| Chlorure de N - dodécyl - N,N - diméthyl - éthyl - benzyl | 0,5-10 |
| Acide Hydroxyéthylamine-diméthylène-phosphonique | 1-10 |
| Polydiméthylsiloxane di-quaternaire | 1-10 |
| Autre : auxiliaires, eau | |

11. Formulation de désinfection selon l'une quelconque des revendications 1 à 7, composée des substances suivantes :
| | % en |
|---|---|
| Dipropylène glycol | 1-10 |
| Chlorure de dioctyl diméthyl ammonium | 0,5-10 |
| Chlorure de N - dodécyl - N,N - diméthyl - éthyl - benzyl - | 0,5-10 |
| Acide Hydroxyéthylamine-diméthylène-phosphonique | 1-10 |
| Polydiméthylsiloxane di-quaternaire | 1-10 |
| Autre : auxiliaires, eau | |

12. Formulation de désinfection selon l'une quelconque des revendications 1 à 7, composée des substances suivantes :
| | % en |
|---|---|
| Dipropylène glycol | 1-10 |
| Alkyl-bis-propylène-triamin | 0,5-5 |
| Chlorure de dioctyldiméthylammonium | 0,5-10 |
| Chlorure de N - dodécyl - N,N - diméthyl - éthyl - benzyl - | 0,5-10 |
| Acide Hydroxyéthylamine-diméthylène-phosphonique | 1-10 |
| Polydiméthylsiloxane di-quaternaire | 1-10 |
| Autre: auxiliaires, eau | |
